# EUROPEAN PATENT SPECIFICATION

(11) **EP 3 158 909 B1**
(45) Date of publication and mention of the grant of the patent: **13.02.2019**
(21) Application number: 15809885.5
(22) Date of filing: 28.04.2015
(51) Int. Cl.: A61B 1/00

(54) **GUIDING DEVICE AND SURGICAL SYSTEM**
FÜHRUNGSVORRICHTUNG UND CHIRURGISCHES SYSTEM
APPAREIL DE GUIDAGE ET SYSTÈME DE CHIRURGIE

(30) Priority: 17.06.2014 JP 2014123868
(43) Date of publication of application: 26.04.2017
(73) Proprietor: OLYMPUS CORPORATION, Hachioji-shi Tokyo 192-8507 (JP)
(72) Inventor: YANAGIHARA, Masaru, Hachioji-shi Tokyo 192-8507 (JP); OGAWA, Ryohei, Hachioji-shi Tokyo 192-8507 (JP); KISHI, Kosuke, Hachioji-shi Tokyo 192-8507 (JP)
(74) Representative: Schicker, Silvia
(86) International application number: PCT/JP2015/062880
(87) International publication number: WO 2015/194263

(56) References cited:
- EP-A2- 1 782 744
- WO-A1-2012/164978
- JP-A- S5 668 426
- JP-A- 2006 158 971
- JP-A- 2010 220 665
- JP-A- 2010 234 058

## Description

### {Technical Field}

The present invention relates to guiding devices and surgical systems, and particularly, to a guiding device having a channel for a medical device, such as an endoscope or a treatment tool, and to a surgical system equipped with the guiding device.

### {Background Art}

A known endoscope in the related art includes a flexible section having a bending part at the distal end thereof, a manipulation unit connected to the proximal end of the flexible section and used for manually operating the bending part, and a treatment-tool channel tube provided within the flexible section (for example, see Patent Literature 1).

### {Citation List}

### {Patent Literature}

{PTL 1}
Japanese Unexamined Patent Application, Publication No. 2010-220665

Document EP1782744, which forms the basis for the preamble of claim 1, discloses another endoscope with a tool rotatable within the shaft of the endoscope.

### {Summary of Invention}

### {Technical Problem}

During surgery, the flexible section is often twisted about its longitudinal axis, and the distal end of the flexible section disposed within the body thus has to be frequently rotated. In this case, it is necessary to apply a large torque to the flexible section against the torsional rigidity of the flexible section. In order to achieve this, the left hand holding the manipulation unit is moved by a large amount so as to rotate the manipulation unit about the flexible section, thereby applying a large torsion to the entire flexible section. This operation is difficult and requires skill, and also involves repetitive large movements, which is problematic in that there is a large physical load on the surgeon.

The present invention has been made in view of the circumstances described above, and an object thereof is to provide a guiding device that allows the distal end of a flexible section to be easily rotated without requiring a large movement or a large force, and a surgical system equipped with the guiding device.

### {Solution to Problem}

In order to achieve the aforementioned object, the present invention provides the following solutions.

The invention is defined in independent claim 1.

A medical device is inserted from the proximal end of the channel tube in a state where the flexible section is inserted in the body, so that the medical device can be guided into the body via the channel tube. Furthermore, by holding the manipulation unit with one hand and manually operating the manipulation unit, the bending direction and the bending angle of the bending part provided at the distal end of the flexible section can be changed, whereby the orientation of the distal end surface of the flexible section from which the medical device protrudes can be changed. Moreover, by holding a part of the flexible section located outside the body with the other hand and twisting the flexible section about the longitudinal axis of the flexible section, the distal end of the flexible section can be rotated, whereby the orientation of the medical device can be changed.

When rotating the distal end of the flexible section, if a torque is applied to the first part by twisting the first part of the flexible section, the proximal end of the first part rotates at the rotating section in accordance with the torque, and the torque is not transmitted to the second part of the flexible section or the manipulation unit. Specifically, the section that generates a resistance force against a twisting operation performed by the operator is only the first part at the rotating section, and a portion of torsional deformation occurring in this first part is canceled out by rotation of the proximal end of the first part. Therefore, the resistance force against the twisting operation performed on the flexible section by the operator is small, and the operator can rotate the distal end of the flexible section by simply applying a small torque thereto. Furthermore, when twisting the flexible section, it is not necessary to move the manipulation unit held with one hand, and the distal end of the flexible section can be easily rotated with only a small movement that involves twisting a section of the first part with the other hand.

The guiding device may further include a rotation support that supports a proximal end of the channel tube in a rotatable manner about a longitudinal axis of the channel tube.

Accordingly, when a torque acts on the channel tube as a result of rotation of the distal end of the flexible section, the proximal end of the channel tube rotates at the rotation support in accordance with this torque. Accordingly, the channel tube can be prevented from twisting, and the resistance force against the twisting operation of the flexible section can be further reduced.

The guiding device may further include a tube holder that is provided within the flexible section and that maintains the channel tube at a position coaxial with the flexible section.

Accordingly, when the flexible section is twisted, the channel tube is twisted at a fixed position relative to the flexible section and is prevented from moving within the flexible section. This can prevent the channel tube from interfering with other components contained in the flexible section.

The guiding device may further include a plurality of the channel tubes that are arranged parallel to each other.

Accordingly, a plurality of medical devices can be simultaneously guided into the body cavity. Moreover, although the overall torsional rigidity of the flexible section increases with an increasing number of components contained in the flexible section, the distal end of the flexible section can be rotated by simply applying a small torque thereto even when the distal end of the flexible section has such large torsional rigidity.

The guiding device may further include a branch section that is provided at the second part, extends in a direction intersecting a longitudinal axis of the second part, and has an insertion port through which a medical device is insertable. A proximal end of the channel tube may be disposed at the insertion port of the branch section.

Accordingly, the medical device can be inserted into the channel tube through the insertion port provided at the branch section.

The branch section may have the insertion port on an extension of a longitudinal axis of the first part, and a proximal end portion of the channel tube may be disposed at the branch section in line with the extension of the longitudinal axis of the first part.

Accordingly, friction occurring between the inner wall of the channel tube and the medical device within the channel tube is reduced, as compared with a configuration in which the proximal end portion of the channel tube is bent at the position of the branch section, thereby allowing for improved ease of operation of the medical device.

The guiding device may further include: an arm that protrudes from the distal end of the flexible section and that has an arm bending part, which is bendable, and an arm channel communicating with the channel tube; and an arm manipulation unit to be operated for bending the arm bending part.

Accordingly, a treatment tool protruding via the channel tube and the arm channel can be finely manipulated by bending the arm bending part.

Further, there is provided a surgical system including the above-described guiding device, a treatment tool insertable into the channel tube of the guiding device, a manipulation input device to be operated by an operator, and a drive controller that electrically drives the treatment tool within the channel tube in accordance with an operation input to the manipulation input device.

### {Advantageous Effects of Invention}

The present invention is advantageous in that the distal end of a flexible section can be easily rotated without requiring a large movement or a large force.

### {Brief Description of Drawings}

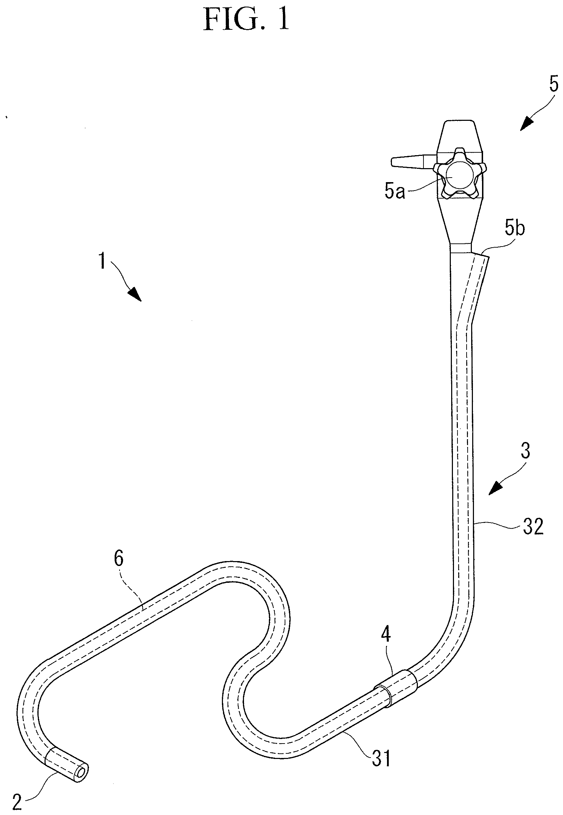

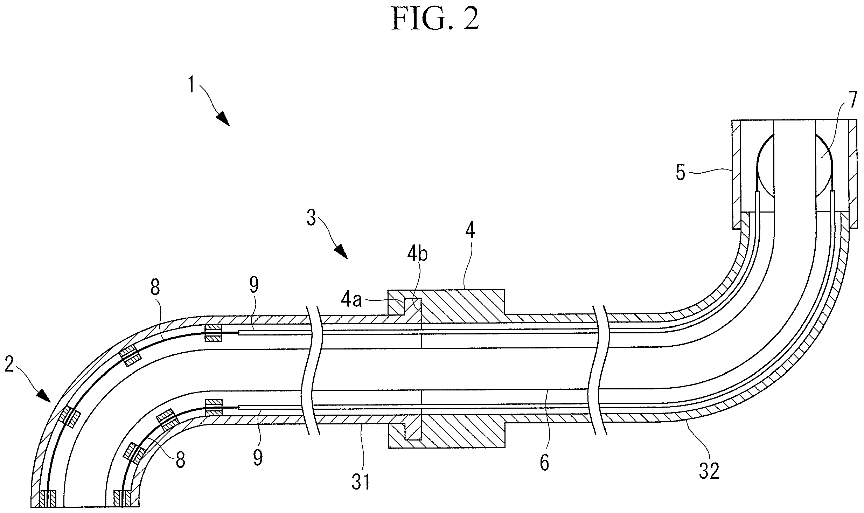

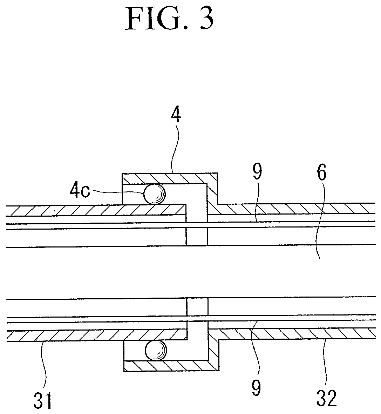

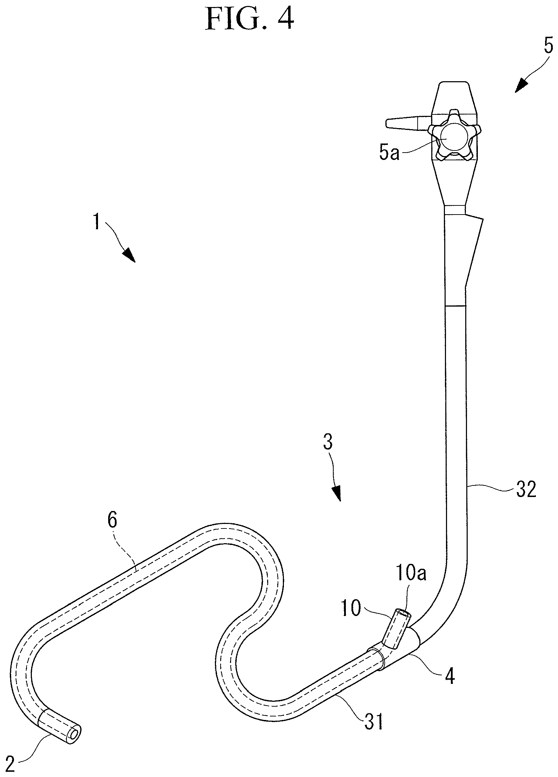

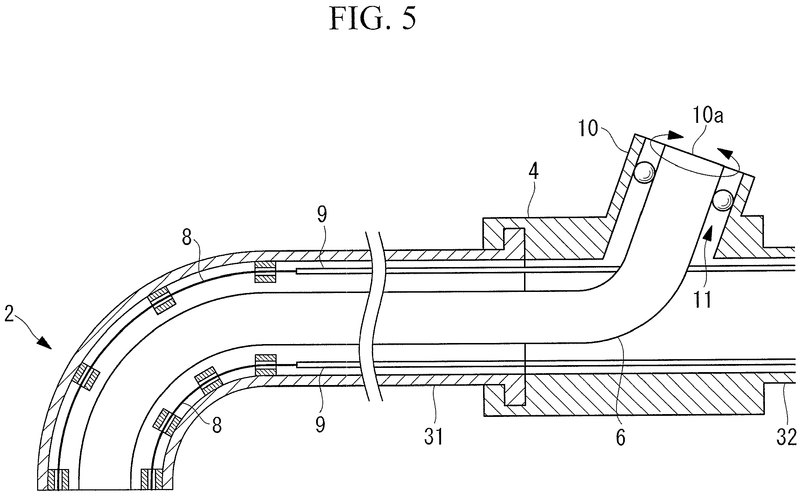

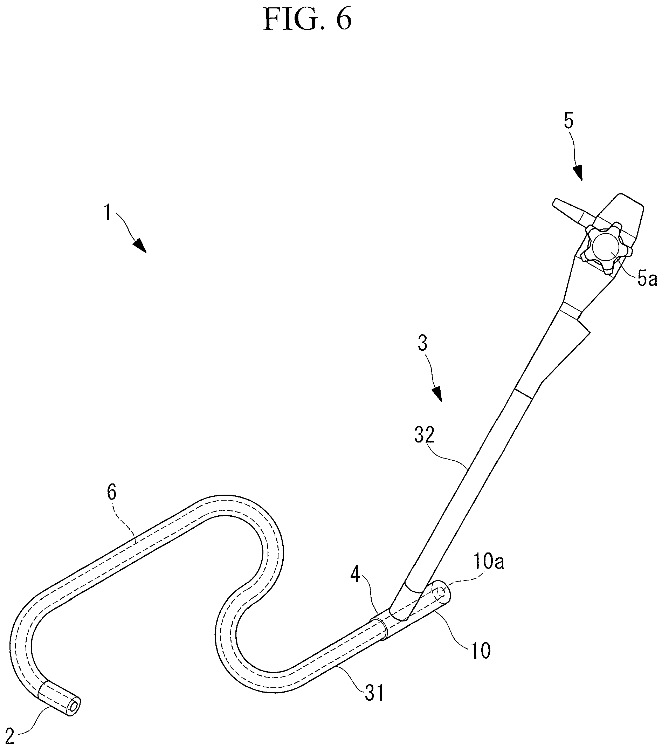

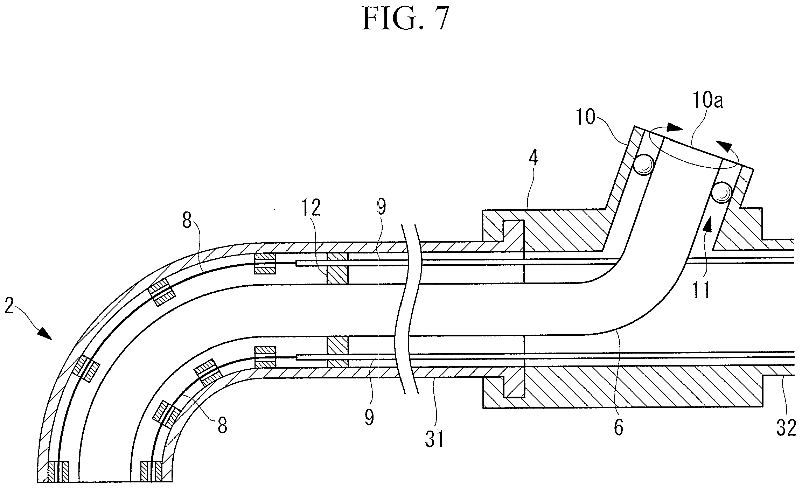

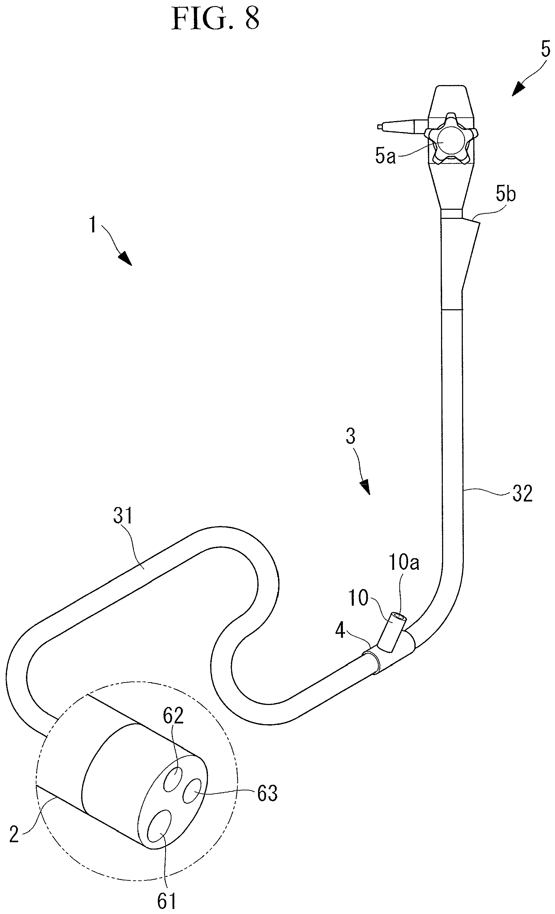

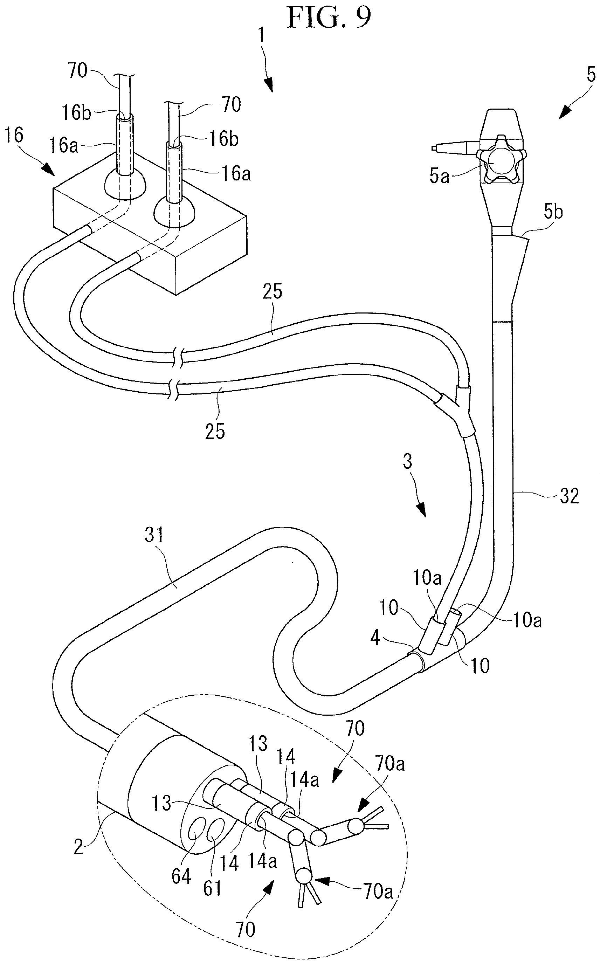

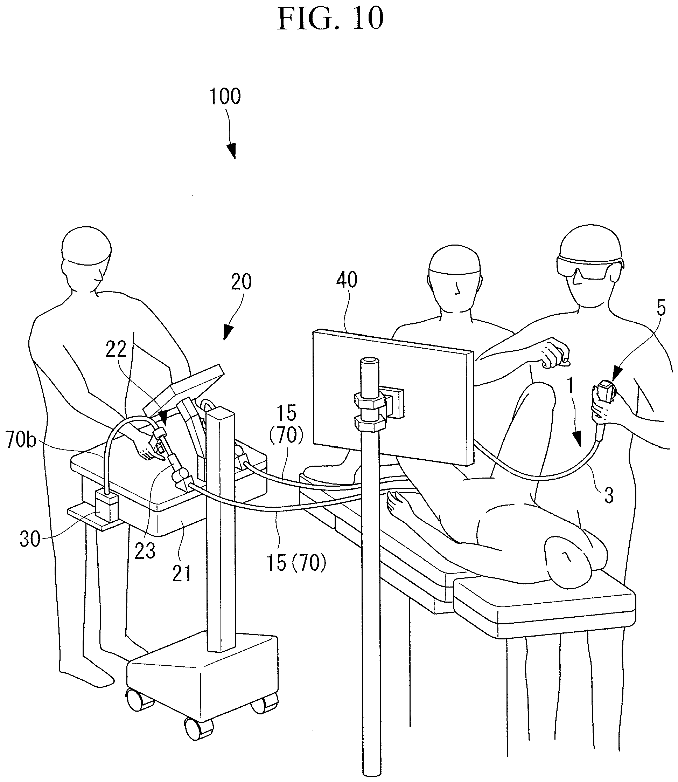

{Fig. 1} Fig. 1 is an external view of a guiding device according to an embodiment of the present invention.
{Fig. 2} Fig. 2 is a vertical sectional view illustrating the internal structure of the guiding device in Fig. 1.
{Fig. 3} Fig. 3 is a vertical sectional view illustrating a modification of a rotating section of the guiding device in Fig. 1.
{Fig. 4} Fig. 4 is an external view of a modification of the guiding device in Fig. 1.
{Fig. 5} Fig. 5 is a vertical sectional view illustrating the configuration of a rotation support provided in a branch section in Fig. 4.
{Fig. 6} Fig. 6 is an external view of another modification of the guiding device in Fig. 1.
{Fig. 7} Fig. 7 is a partial vertical sectional view illustrating another modification of the guiding device in Fig. 1.
{Fig. 8} Fig. 8 is an external view of another modification of the guiding device in Fig. 1.
{Fig. 9} Fig. 9 is an external view of another modification of the guiding device in Fig. 1.
{Fig. 10} Fig. 10 illustrates the overall configuration of a surgical system equipped with the guiding device in Fig. 9.

### {Description of Embodiments}

A guiding device 1 according to an embodiment of the present invention will be described below with reference to the drawings.

As shown in Figs. 1 and 2, the guiding device 1 according to this embodiment includes an elongate flexible section 3 having a bending part 2 at the distal end thereof, a rotating section 4 provided at an intermediate position of the flexible section 3 in the longitudinal direction thereof, a manipulation unit 5 connected to the proximal end of the flexible section 3, and a channel tube 6 disposed in the longitudinal direction within the flexible section 3.

The flexible section 3 includes flexible tubes 31 and 32, which have flexibility and cover the outer periphery of the flexible section 3, and, over the entirety thereof, is flexible and bendable in conformity to the shape of the body cavity. The flexible tubes 31 and 32 are two parts that are separated from each other at an intermediate position of the flexible section 3 in the longitudinal direction and includes a first part 31 that is located at the distal end and that is to be inserted into the body cavity and a second part 32 that is located at the proximal end and that is to be disposed outside the body. The lengths of the first part 31 and the second part 32 can be appropriately selected. For example, if the guiding device 1 is to be used for treatment of appendicitis, the length of the first part 31 is 1000 mm, and the length of the second part 32 is 600 mm.

The rotating section 4 connects the first part 31 and the second part 32 to each other in a manner allowing relative rotation thereof about the longitudinal axis of the flexible section 3. In detail, the outer peripheral surface of the proximal end portion of the first part 31 is provided with a flange-like protrusion 4a extending in the circumferential direction. The inner peripheral surface of the distal end portion of the second part 32 is provided with an annular guide groove 4b extending in the circumferential direction. The rotating section 4 is configured by engaging the distal end portion of the second part 32 with the outer side of the proximal end portion of the first part 31 such that the protrusion 4a fits into the guide groove 4b.

Alternatively, as shown in Fig. 3, the rotating section 4 may be provided with a rolling bearing 4c that is disposed in an annular gap between the outer peripheral surface of the proximal end portion of the first part 31 and the inner peripheral surface of the distal end portion of the second part 32 and that supports the proximal end portion of the first part 31 and the distal end portion of the second part 32 in a manner allowing relative rotation thereof.

Furthermore, the connection area between the first part 31 and the second part 32 may be provided with a waterproof seal (not shown) for preventing water from entering the guiding device 1 through the gap between the first part 31 and the second part 32 when, for example, cleaning the guiding device 1.

The manipulation unit 5 includes an angle knob 5a to be used by an operator for manually controlling the bending direction and the bending angle of the bending part 2. Reference sign 7 denotes a pulley that rotates together with the angle knob 5a, and reference sign 8 denotes a manipulation wire that connects the pulley 7 and the distal end of the flexible section 3. The outer peripheral surface of the manipulation wire 8 is covered with a sheath 9. When the angle knob 5a rotates, the pulley 7 rotates so as to push and pull the manipulation wire 8, thereby bending the bending part 2.

The channel tube 6 has an inner diameter that allows a medical device, such as an endoscope or a treatment tool, to be inserted therein, and has openings at both ends thereof. The distal end of the channel tube 6 is fixed to the distal end surface of the flexible section 3. The proximal end of the channel tube 6 is disposed at an insertion port 5b provided in the manipulation unit 5. The channel tube 6 has higher flexibility than the flexible tubes 31 and 32 of the flexible section 3 and is deformable in conformity to bending and twisting of the flexible section 3 and the bending part 2.

Next, the operation of the guiding device 1 having the above-described configuration will be described.

The guiding device 1 according to this embodiment is used in surgery that involves the use of an endoscope or a treatment tool. For example, the first part 31 of the flexible section 3 is inserted into the large intestine through the anus of a patient so that the distal end of the flexible section 3 is disposed in a target area within the large intestine. Then, an endoscope is inserted into the channel tube 6 through the insertion port 5b of the manipulation unit 5 so that the endoscope protrudes from an opening in the distal end surface of the flexible section 3. Thus, the interior of the large intestine can be observed with the endoscope.

The operator rotates the angle knob 5a of the manipulation unit 5 held with his/her left hand so as to bend the bending part 2. This causes the distal end of the endoscope to be oriented toward the inner wall of the large intestine, so that the inner wall can be observed. Moreover, with the bending part 2 in the bent state, the operator holds a section of the first part 31 exposed outside the body by the operator's right hand and twists this section about the longitudinal axis so as to rotate the distal end of the flexible section 3. This causes the distal end of the endoscope to move in the circumferential direction along the inner wall of the large intestine, so that the observation range of the inner wall can be moved in the circumferential direction.

When twisting the flexible section 3, the operator feels a resistance force caused by torsional rigidity of the flexible section 3. This resistance force increases with an increasing amount of torsion of the flexible section 3. Among the components constituting the flexible section 3, the torsional rigidities of the flexible tubes 31 and 32 are especially high. In this embodiment, the first part 31 and the second part 32 are connected to each other in a manner allowing relative rotation thereof by the rotating section 4 so that even when the first part 31 is twisted, the torsion is not transmitted to the second part 32 or the manipulation unit 5. Specifically, the section that generates a resistance force against a twisting operation performed by a surgeon is mainly the first part 31. Furthermore, since torsional deformation occurring in the first part 31 is cancelled out by the rotation of the distal end and the proximal end thereof, the amount of torsion and the resistance force do not increase. This is advantageous in that the operator can easily rotate the distal end of the flexible section 3 by simply applying a small torque by performing a small twisting movement with his/her right hand.

In this embodiment, the opening at the proximal end of the channel tube 6 is disposed at the insertion port 5b of the manipulation unit 5. Alternatively, as shown in Fig. 4, the distal end portion of the second part 32 may be provided with a branch section 10, and the opening at the proximal end of the channel tube 6 may be disposed at an insertion port 10a provided in the branch section 10. The branch section 10 extends in a direction intersecting the longitudinal axis of the second part 32 and has the insertion port 10a at the distal end thereof.

Accordingly, this allows for improved user-friendliness in a case where the guiding device 1 and the medical device are to be operated by different operators.

Furthermore, in this embodiment, a rotation support that supports the proximal end of the channel tube 6 in a rotatable manner about the longitudinal axis of the channel tube 6 may be provided.

For example, as shown in Fig. 5, in the configuration equipped with the branch section 10, the branch section 10 may be provided with a rotation support 11. The rotation support 11 is constituted of, for example, a rolling bearing that supports the outer peripheral surface of the proximal end portion of the channel tube 6. The branch section 10 may be provided with a waterproof seal (not shown) for preventing water from entering from the periphery of the channel tube 6.

Although the channel tube 6 also becomes twisted when the first part 31 is twisted, this torsion of the channel tube 6 is quickly canceled out by rotation of the proximal end portion thereof supported by the rotation support 11. Therefore, the channel tube 6 can be prevented from being twisted by a large amount, thus preventing the channel tube 6 from being crushed at an intermediate position thereof. Furthermore, since the torsion of the channel tube 6 is reduced, a resistance force against the twisting operation performed on the flexible section 3 can be further reduced.

As shown in Fig. 6, the branch section 10 may have the insertion port 10a on the extension of the longitudinal axis of the first part 31 with respect to the proximal end of the first part 31, that is, at a position opposed to the first part 31 in the direction of the longitudinal axis thereof, such that the channel tube 6 extends straight from the proximal end of the first part 31 to the insertion port 10a. In this case, the second part 32 is connected to the first part 31 at a certain angle instead of being connected straight thereto.

In a bent area of the channel tube 6, friction tends to occur due to contact between the inner wall of the channel tube 6 and the medical device. This friction leads to reduced ease of operation of the medical device. By providing the insertion port 10a at a position that allows the proximal end portion of the channel tube 6 to be disposed straight, the friction occurring between the channel tube 6 and the medical device can be reduced, thereby allowing for improved ease of operation of the medical device.

Furthermore, as shown in Fig. 7, in this embodiment, a tube holder 12 that maintains the channel tube 6 at a position coaxial with the flexible section 3 is preferably provided within the flexible section 3. The tube holder 12 is, for example, a disk-shaped member disposed in the radial direction within the flexible section 3. This disk-shaped member has its outer peripheral surface fixed to the inner peripheral surface of the flexible tubes 31 and 32 and has a hole in the center through which the channel tube 6 extends. A plurality of tube holders 12 may be provided at a plurality of positions in the longitudinal direction of the flexible section 3.

In a case where the longitudinal axis of the channel tube 6 is decentered from the longitudinal axis of the flexible section 3, the channel tube 6 may possibly rotate about the longitudinal axis of the flexible section 3 and interfere with surrounding components. In contrast, in the case where the longitudinal axis of the channel tube 6 is aligned with the longitudinal axis of the flexible section 3, the channel tube 6 rotates about the longitudinal axis at a fixed position relative to the flexible section 3. Therefore, the channel tube 6 can be prevented from interfering with surrounding components.

Furthermore, although a single-lumen-type guiding device 1 equipped with a single channel tube 6 is described in this embodiment, the guiding device 1 may be of a multi-lumen-type that has a plurality of channel tubes 61, 62, and 63 arranged parallel to one another and that is capable of simultaneously guiding a plurality of medical devices, as shown in Fig. 8. For example, the guiding device 1 may have a single endoscope channel tube 61 and two treatment-tool channel tubes 62 and 63. The opening at the proximal end of each of the channel tubes 61, 62, and 63 may be disposed at the insertion port of one of the manipulation unit 5 and the branch section 10.

The overall torsional rigidity of the flexible section 3 increases with an increasing number of components contained in the flexible section 3. According to this embodiment, even with the distal end of the flexible section 3 having such large torsional rigidity, the distal end of the flexible section 3 can be rotated with only a small movement and a small force.

Furthermore, as shown in Fig. 9, in this embodiment, the guiding device 1 may include a pair of left and right arms 14 each having a manually-operable or electrically-operable arm bending part 13. Reference sign 16 denotes an arm manipulation unit to be operated for bending the arm bending parts 13. The arm manipulation unit 16 is provided with manipulation members 16a, such as levers or joysticks. When the manipulation members 16a are operated by the operator, the arm bending parts 13 are bent and driven in accordance with the operation.

The arms 14 protrude from the distal end surface of the flexible section 3 and individually have arm channels 14a that communicate with the treatment-tool channel tubes. The arm channels 14a respectively communicate with insertion ports 16b provided in the manipulation members 16a via channel tubes 25 extending from the branch section 10. By changing the bending direction of the arm bending parts 13 by operating the manipulation members 16a in a state where, for example, treatment tools 70 are inserted in the arm channels 14a through the insertion ports 16b, the orientation of, for example, the treatment tools 70 protruding from the arm channels 14a can be changed.

The distal end surface of the guiding device 1 is provided with a channel tube 64 that communicates with the insertion port 5b, so that an auxiliary treatment tool can be inserted into the body from the insertion port 5b via the channel tube 64. Furthermore, the channel tube 61 communicates with the insertion port 10a so that an endoscope, for example, can be inserted into the body from the insertion port 10a via the channel tube 61.

The guiding device 1 in Fig. 9 can be applied to a surgical system 100 shown in Fig. 10.

The surgical system 100 includes the guiding device 1, a manipulation input device 20 to be operated by an operator, a drive controller 30 that electrically drives the arm bending parts 13 and the treatment tools 70 within the treatment-tool channel tubes of the guiding device 1 in accordance with an operation input to the manipulation input device 20, and a display unit 40 that displays an endoscope image acquired by the endoscope within the endoscope channel tube.

In this guiding device 1, the proximal ends of the two treatment-tool channel tubes are disposed at the insertion port 10a of the branch section 10. The proximal ends of channel tubes 15 extending outward from the insertion port 10a are connected to manipulation members 23, such as levers or joysticks.

The manipulation input device 20 includes a manipulation table 21 set beside a bed on which a patient lies and the manipulation members 23 fixed on the manipulation table 21. The manipulation input device 20 has manipulation units 22 that are inserted through insertion ports (not shown) of the manipulation members 23 and that are attached to flexible sections 70b of the treatment tools 70 having distal-end joints 70a, and transmit an input signal corresponding to an operation input from the manipulation units 22 to the drive controller 30.

The drive controller 30 actuates the distal-end joints 70a of the treatment tools 70 in accordance with the input signal from the manipulation input device 20.

The manipulation members 23 and the channel tubes 15 shown in Fig. 10 respectively correspond to the manipulation members 16a and the channel tubes 25 shown in Fig. 9. Specifically, the configuration shown in Fig. 10 is obtained by detaching the manipulation members 16a shown in Fig. 9 from the base and installing them in the manipulation table 21.

Assuming that the flexible section 3 of the guiding device 1 is not provided with the rotating section 4, the entire flexible section 3 would have to be twisted by a large amount to rotate the distal end of the flexible section 3. In this case, since the proximal ends of the treatment tools 70 extending from the branch section 10 are connected to the drive controller 30 fixed to the manipulation table 21, a large torsion would also occur in the channel tubes 15 and the treatment tools 70 within the channel tubes 15, causing the sections of the channel tubes 15 and the treatment tools 70 located outside the body (i.e., external sections) to move by a large distance or to bend with excessive curvature. If the movement or bending of the external sections poses a problem for electrically operating the treatment tools 70, the operator would have to temporarily suspend the operation of the manipulation input device 20 to correct the positions and shapes of the external sections of the channel tubes 15 and the treatment tools 70.

In contrast, in this embodiment, the flexible sections 3 are provided with the rotating sections 4 so that only a slight torsion occurs in the treatment tools 70. Thus, the external sections of the channel tubes 15 and the treatment tools 70 remain substantially still without being affected by the twisting operation of the flexible sections 3. Accordingly, this is advantageous in that the operator can concentrate on using the manipulation units 22 to electrically operate the treatment tools 70.

### {Reference Signs List}

- 1: guiding device
- 2: bending part
- 3: flexible section
- 4: rotating section
- 4a: protrusion
- 4b: guide groove
- 4c: rolling bearing
- 5: manipulation unit
- 5a: angle knob
- 5b: insertion port
- 6: channel tube
- 7: pulley
- 8: manipulation wire
- 9: sheath
- 10: branch section
- 10a: insertion port
- 11: rotation support
- 12: tube holder
- 13: arm bending part
- 14: arm
- 14a: arm channel
- 15: channel tube
- 16: arm manipulation unit
- 20: manipulation input device
- 21: manipulation table
- 22: manipulation unit
- 30: drive controller
- 31: first part
- 32: second part
- 40: display unit
- 100: surgical system

## Claims

1. A guiding device (1) comprising:
an elongate flexible section (3) having flexibility and provided with a bending part (2), which is bendable, at a distal end thereof;
a channel tube (6) having flexibility and disposed in a longitudinal direction within the flexible section; and
a manipulation unit (5) that is provided at a proximal end of the flexible section and that is to be manually operated for bending the bending part,
wherein the flexible section has a first part at the distal end and a second part at the proximal end,
**characterized by** a rotating section (4) provided at an intermediate position of the flexible section in the longitudinal direction, wherein the rotating section connects the first part to the second part in a rotatable manner about a longitudinal axis of the flexible section.

2. The guiding device according to Claim 1, further comprising:
a rotation support (11) that supports a proximal end of the channel tube in a rotatable manner about a longitudinal axis of the channel tube.

3. The guiding device according to Claim 1 or 2, further comprising:
a tube holder (12) that is provided within the flexible section and that maintains the channel tube at a position coaxial with the flexible section.

4. The guiding device according to any one of Claims 1 to 3, further comprising: a plurality of the channel tubes (15) that are arranged parallel to each other.

5. The guiding device according to any one of Claims 1 to 4, further comprising:
a branch section (10) that is provided at the second part, extends in a direction intersecting a longitudinal axis of the second part, and has an insertion port through which a medical device is insertable,
wherein a proximal end of the channel tube is disposed at the insertion port of the branch section.

6. The guiding device according to Claim 5,
wherein the branch section (10) has the insertion port (10a) on an extension of a longitudinal axis of the first part, and
wherein a proximal end portion of the channel tube (15) is disposed at the branch section in line with the extension of the longitudinal axis of the first part.

7. The guiding device according to any one of Claims 1 to 6, further comprising:
an arm (14) that protrudes from the distal end of the flexible section and that has an arm bending part (13), which is bendable, and an arm channel communicating with the channel tube; and
an arm manipulation unit (16) to be operated for bending the arm bending part.

8. A surgical system comprising:
the guiding device according to any one of Claims 1 to 7;
a treatment tool insertable into the channel tube of the guiding device;
a manipulation input device (20) to be operated by an operator; and
a drive controller (30) that electrically drives the treatment tool within the channel tube in accordance with an operation input to the manipulation input device.

## Patentansprüche

1. Führungsvorrichtung (1), umfassend:
einen länglichen flexiblen Abschnitt (3), der Flexibilität aufweist und an einem distalen Ende davon mit einem Biegeteil (2) versehen ist, das biegbar ist;
einen Kanalschlauch (6), der Flexibilität aufweist und in einer Längsrichtung innerhalb des flexiblen Bereichs angeordnet ist; und
eine Manipulationseinheit (5), die an einem proximalen Ende des flexiblen Abschnitts vorgesehen ist und die manuell zu bedienen ist, um das Biegeteil zu biegen,
wobei der flexible Abschnitt ein erstes Teil an dem distalen Ende und ein zweites Teil an dem proximalen Ende aufweist,
**gekennzeichnet durch**
einen Drehabschnitt (4), der in einer Zwischenposition des flexiblen Abschnitts in der Längsrichtung vorgesehen ist, wobei der Drehabschnitt das erste Teil mit dem zweiten Teil drehbar um eine Längsachse des flexiblen Abschnitts verbindet.

2. Führungsvorrichtung nach Anspruch 1, ferner umfassend:
eine Drehstütze (11), die ein proximales Ende des Kanalschlauchs um eine Längsachse des Kanalschlauchs drehbar stützt.

3. Führungsvorrichtung nach Anspruch 1 oder 2, ferner umfassend:
eine Schlauchhalterung (12), die innerhalb des flexiblen Abschnitts vorgesehen ist und die den Kanalschlauch in einer Position koaxial zu dem flexiblen Abschnitt hält.

4. Führungsvorrichtung nach einem der Ansprüche 1 bis 3, ferner umfassend:
eine Mehrzahl von Kanalschläuchen (15), die parallel zueinander angeordnet sind.

5. Führungsvorrichtung nach einem der Ansprüche 1 bis 4, ferner umfassend:
einen Verzweigungsabschnitt (10), der an dem zweiten Teil vorgesehen ist, sich in eine Richtung erstreckt, die eine Längsachse des zweiten Teils kreuzt, und eine Einführöffnung aufweist, durch die eine medizinische Vorrichtung einführbar ist,
wobei ein proximales Ende des Kanalschlauchs an der Einführöffnung des Verzweigungsabschnitts angeordnet ist.

6. Führungsvorrichtung nach Anspruch 5,
wobei der Verzweigungsabschnitt (10) die Einführöffnung (10a) an einer Verlängerung einer Längsachse des ersten Teils aufweist, und
wobei ein proximaler Endabschnitt des Kanalschlauchs (15) an dem Verzweigungsabschnitt in Übereinstimmung mit der Verlängerung der Längsachse des ersten Teils angeordnet ist.

7. Führungsvorrichtung nach einem der Ansprüche 1 bis 6;
ferner umfassend:
einen Arm (14), der von dem distalen Ende des flexiblen Abschnitts vorspringt und ein Armbiegeteil (13) aufweist, das biegbar ist, und
einen Armkanal, der mit dem Kanalschlauch kommuniziert; und
eine Armmanipulationseinheit (16), die zu bedienen ist, um das Armbiegeteil zu biegen.

8. Chirurgisches System, umfassend:
die Führungsvorrichtung nach einem der Ansprüche 1 bis 7;
ein Behandlungswerkzeug, das in den Kanalschlauch der Führungsvorrichtung einführbar ist;
eine Manipulationseingabeeinrichtung (20), die von einem Bediener zu bedienen ist; und
eine Antriebssteuerung (30), die das Behandlungswerkzeug innerhalb des Kanalschlauchs in Übereinstimmung mit einer Bedienungseingabe in die Manipulationseingabeeinrichtung elektrisch antreibt.

## Revendications

1. Dispositif de guidage (1) comprenant :
une section souple allongée (3) ayant une flexibilité et comportant une partie de courbure (2), qui peut être courbée, à une extrémité distale de celle-ci ;
un tube de canal (6) ayant une flexibilité et disposé dans une direction longitudinale à l'intérieur de la section souple ; et
une unité de manipulation (5) qui est prévue à une extrémité proximale de la section souple et qui doit être actionnée manuellement pour courber la partie de courbure,
la section souple ayant une première partie à l'extrémité distale et une seconde partie à l'extrémité proximale,
**caractérisé par** une section de rotation (4) prévue à une position intermédiaire de la section souple dans la direction longitudinale, la section de rotation reliant la première partie à la seconde partie d'une manière rotative autour d'un axe longitudinal de la section souple.

2. Dispositif de guidage selon la revendication 1, comprenant en outre :
un support de rotation (11) qui supporte une extrémité distale du tube de canal d'une manière rotative autour d'un axe longitudinal du tube de canal.

3. Dispositif de guidage selon la revendication 1 ou 2, comprenant en outre :
un support de tube (12) qui est prévu à l'intérieur de la section souple et qui maintient le tube de canal à une position coaxiale à la section souple.

4. Dispositif de guidage selon l'une quelconque des revendications 1 à 3, comprenant en outre :
une pluralité de tubes de canal (15) qui sont disposés parallèlement les uns aux autres.

5. Dispositif de guidage selon l'une quelconque des revendications 1 à 4, comprenant en outre :
une section de ramification (10) qui est prévue à la seconde partie, s'étend dans une direction coupant un axe longitudinal de la seconde partie, et a un orifice d'introduction à travers lequel un dispositif médical peut être introduit,
une extrémité proximale du tube de canal étant disposée à l'orifice d'introduction de la section de ramification.

6. Dispositif de guidage selon la revendication 5,
dans lequel la section de ramification (10) a l'orifice d'introduction (10a) sur une extension d'un axe longitudinal de la première partie, et
dans lequel une partie d'extrémité proximale du tube de canal (15) est disposée à la section de ramification en alignement avec l'extension de l'axe longitudinal de la première partie.

7. Dispositif de guidage selon l'une quelconque des revendications 1 à 6, comprenant en outre :
un bras (14) qui fait saillie à partir de l'extrémité distale de la section souple et qui a une partie de courbure de bras (13), qui peut être courbée, et un canal de bras en communication avec le tube de canal ; et
une unité de manipulation de bras (16) à actionner pour courber la partie de courbure de bras.

8. Système chirurgical comprenant :
le dispositif de guidage selon l'une quelconque des revendications 1 à 7 ;
un outil de traitement apte à être introduit dans le tube de canal du dispositif de guidage ;
un dispositif d'entrée de manipulation (20) à actionner par un opérateur ; et
un dispositif de commande d'entraînement (30) qui entraîne électriquement l'outil de traitement à l'intérieur du tube de canal conformément à une entrée d'actionnement dans le dispositif d'entrée de manipulation.
